# EUROPEAN PATENT APPLICATION

(11) **EP 4 325 512 A1**
(43) Date of publication of application: **21.02.2024**
(21) Application number: 23775368.6
(22) Date of filing: 27.03.2023
(51) Int. Cl.: G16H 20/60, G16H 10/60, G16H 10/40, G06N 3/08

(54) **ARTIFICIAL INTELLIGENCE-BASED MEAL MONITORING METHOD AND APPARATUS**

(30) Priority: 25.03.2022 KR 20220037529
(71) Applicant: Nuvi Labs Co., Ltd., Seoul 06173 (KR)
(72) Inventor: RU, Jey Yoon, Seoul 06173 (KR); KIM, Dae Hoon, Seoul 06173 (KR); LUCA, Pimenta Medeiros, Seoul 06173 (KR)
(74) Representative: Habermann, Hruschka & Schnabel
(86) International application number: PCT/KR2023/004000
(87) International publication number: WO 2023/182873

(57) **Abstract**

The present invention relates to a meal monitoring method and apparatus. The meal monitoring method according to an embodiment of the present invention comprises the steps of: generating food information by analyzing a pre-stored menu; obtaining an image including a food tray on the basis of at least one photographing apparatus; classifying at least one dish included in the captured image on the basis of the generated food information; and generating and storing meal information on the basis of the classified at least one dish, wherein the generating of the food information may comprise: extracting a food list from the pre-stored menu; sub-dividing the extracted food list on the basis of a pre-stored classification table; and generating the sub-divided food list as the food information.

## Description

### [Technical Field]

The present disclosure relates to artificial intelligence-based meal monitoring method and apparatus.

### [Background Art]

Recently, people's interest in health has been increasing; on the other hand, the number of people suffering from overweight or obesity is also gradually increasing. Overweight or obesity is a serious problem that causes various diseases such as diabetes and high blood pressure.

Therefore, to solve overweight or obesity, it is necessary to analyze one's eating habits first. In general, people know their likes and dislikes but do not remember the types of objects and how often they actually eat. Therefore, to analyze one's eating habits, it is necessary to identify the objects actually consumed and analyze the individual's eating habits based on the information on the identified objects.

However, to determine the food actually consumed, the user has to enter food information using a mobile apparatus, which increases user inconvenience and reduces satisfaction with a service that analyzes an individual's eating habits.

To address the problem, while methods are being developed to automatically recognize food based on images captured either automatically or manually, automatically generate food information, and subsequently record the generated food information as meal information, it is challenging to automatically recognize food solely with images.

Therefore, there is a need to develop a method that more accurately recognizes or classifies food a user has consumed based on images captured either automatically or manually and enables to record highly reliable meal information.

### [Disclosure]

### [Technical Problem]

To solve the problem above, the present disclosure provides a meal monitoring method and apparatus that more accurately recognize or classify food a user has consumed based on images captured either automatically or manually and enable to record highly reliable meal information.

Also, the present disclosure provides a meal monitoring method and apparatus capable of preventing an unnecessary search when analyzing images captured automatically or manually by subdividing food items on a food menu into the foods a user may consume and including even those not explicitly listed on the food menu.

The technical objects of the present disclosure are not limited to those described above, and other technical objects not mentioned above may be understood clearly by those skilled in the art from the descriptions given below.

### [Technical Solution]

To solve the problem above, a meal monitoring method according to one embodiment of the present disclosure may comprise generating food information by analyzing a pre-stored food menu; obtaining an image including a food tray based on at least one image capture apparatus; classifying at least one food included in the captured image based on the generated food information; and generating and storing food information based on the classified at least one food, wherein the generating of the food information may comprise extracting a food list from the pre-stored food menu; subdividing the extracted food list based on a pre-stored classification table; and generating the subdivided food list as food information.

Meanwhile, a meal monitoring apparatus according to one embodiment of the present disclosure may comprise a communication module; an image capture module capturing an image including a food tray based on at least one image capture apparatus; a storage storing various pieces of information or data, and at least one process required to monitor a meal; and a controller performing an operation for monitoring the meal based on the at least one process, wherein the controller generates food information by analyzing a pre-stored food menu; classifies at least one food included in a captured image based on the generated food information when the image including a food tray is obtained based on at least one image capture apparatus; and generates and stores food information based on the classified at least one food, wherein, when the food information is generated, a food list is extracted from the pre-stored food menu; the extracted food list is subdivided based on a pre-stored classification table; and the subdivided food list is generated as food information.

Other specific details of the present disclosure are included in the detailed description and drawings below.

### [Advantageous Effects]

The present disclosure more accurately recognizes or classifies food a user has consumed based on images captured either automatically or manually, thereby enabling to record highly reliable meal information.

Also, the present disclosure may prevent an unnecessary search when analyzing images captured automatically or manually by subdividing food items on a food menu into the foods a user may consume and including even those not explicitly listed on the food menu.

The technical effects of the present disclosure are not limited to the technical effects described above, and other technical effects not mentioned herein may be understood to those skilled in the art to which the present disclosure belongs from the description below.

### [Description of Drawings]

FIG. 1 illustrates one example of capturing a food tray using an image capture apparatus.
FIG. 2 illustrates a network structure of a meal monitoring system according to an embodiment of the present disclosure.
FIG. 3 is a block diagram illustrating the structure of a meal monitoring apparatus according to an embodiment of the present disclosure.
FIG. 4 is a flow diagram illustrating a meal monitoring method according to an embodiment of the present disclosure.
FIG. 5 illustrates a specific operation of generating food information by a meal monitoring method according to an embodiment of the present disclosure.
FIG. 6 illustrates one example of a mix class and a subclass that may be included in a pre-stored classification table according to one embodiment of the present disclosure.
FIG. 7 illustrates the relationship between foods included in a pre-stored food menu and a subdivided food list according to one embodiment of the present disclosure.

### [Mode for Disclosure]

The advantages and features of the present disclosure, and a method for achieving them will be clearly understood with reference to the embodiments described together with appended drawings. However, the present disclosure is not limited to the embodiments disclosed below but may be implemented in various other forms; the present embodiments are provided only to make the present disclosure complete and inform those skilled in the art clearly of the technical scope of the present disclosure, and the present disclosure may be defined only by the technical scope of the appended claims.

The terms used herein are intended to describe embodiments and are not intended to limit the present disclosure. In the present disclosure, a singular expression includes a plural expression unless clearly indicated otherwise in the corresponding phrase. The term "comprises" and/or "comprising" used in the present disclosure indicates the existence of a constituting element, a step, an operation, and/or a component described but does not exclude the existence or addition of one or more other constituting elements, steps, operations, and/or components. Throughout the document, the same drawing symbol indicates the same constituting element, and the term "and/or" includes each individual constituting element mentioned and every possible combination involving one or more of the constituting elements. Although the terms such as "first" and "second" are used to describe various constituting elements, it should be understood that these constituting elements are not limited by those terms. The terms are introduced to distinguish one constituting element from the others. Therefore, without departing from the technical scope of the present disclosure, a first constituting element may be referred to as a second constituting element, and vice versa.

Unless otherwise defined, all terms (including technical and scientific terms) used herein may be used in a sense commonly understood by those skilled in the art to which the present disclosure belongs. Also, terms defined in commonly used dictionaries are not ideally or excessively interpreted unless otherwise explicitly defined.

Terms describing the spatial relationship such as "below," "beneath," "lower," "above," and "upper," as shown in the drawing, may be used to easily describe the spatial relationship between one constituting element and other constituting elements. The spatially relative terms should be understood as those that include different directions of the constituting elements during their use or operation in addition to the directions indicated in the drawings. For example, if a constituting element shown in a drawing is flipped over, the constituting element, which may have been described as "below" or "beneath" another constituting element, may be placed "above" yet another component. Accordingly, the illustrative term "down" may include both downward and upward directions. A constituting element may be oriented to face a different direction; therefore, spatially relative terms may be interpreted differently according to the orientation of the constituting element.

The term "unit" or "module" used in the present disclosure may refer to a software component or a hardware component such as a Field Programmable Gate Array (FPGA) or an Application Specific Integrated Circuit (ASIC), and the "unit" or "module" performs a particular function. However, the "unit" or "module" is not necessarily limited to a software or hardware component. The "unit" or "module" may be configured to be implemented in an addressable storage medium or configured to operate one or more processors. Therefore, for example, the "unit" or "module" includes those components such as software components, object-oriented software components, class components, and task components; processes, functions, properties, procedures, subroutines, segments of a program code, drivers, firmware, micro-code, circuits, data, databases, data structures, tables, arrays, and variables. The functions provided by the constituting elements and the "unit" or "module" of the present disclosure may be combined into a smaller number of constituting elements, "units", and "modules" or further divided into additional constituting elements, "units", or "modules".

Unless otherwise defined, all terms (including technical and scientific terms) used herein may be used in a sense commonly understood by those skilled in the art to which the present disclosure belongs. Also, terms defined in commonly used dictionaries are not ideally or excessively interpreted unless otherwise explicitly defined.

In what follows, embodiments of the present disclosure will be described in detail with reference to appended drawings.

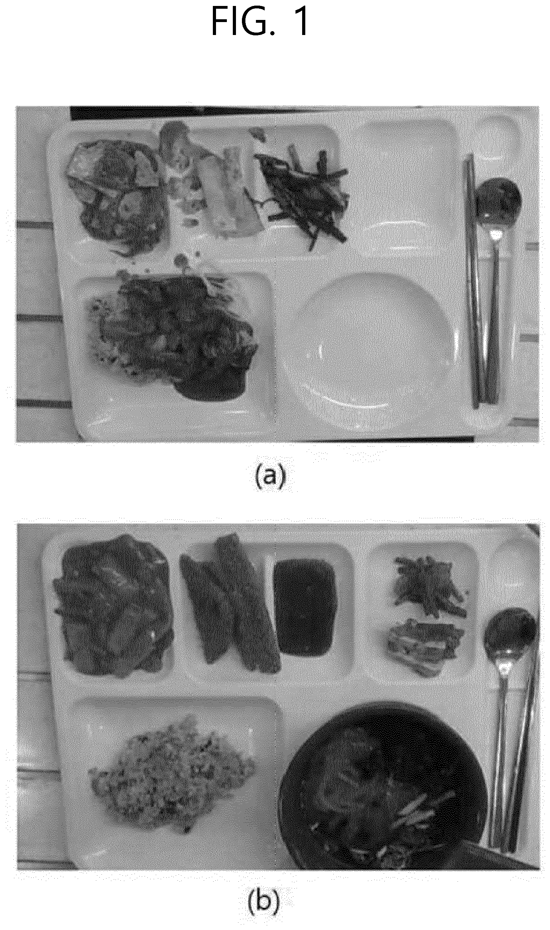

FIG. 1 illustrates one example of capturing a food tray using an image capture apparatus.

To monitor a user's meal, a picture of a food tray (dishes) containing the food the user wants to eat is automatically or manually captured at a cafeteria, a restaurant, or home.

If the user takes a photo and directly enters the food information, there is little chance that a technical problem will occur; however, if food information is automatically generated by analyzing the food the user has consumed through captured images, certain food may not be recognized due to insufficient information, or classification of some foods hard to be classified may result in misclassification.

In the case of a cafeteria, since a food menu is available in advance, it is possible to monitor a user's meals by storing the food menu in advance and using the stored food menu for analysis. However, even in the cafeteria scenario, accurate analysis may not be attained since the analysis result may vary depending on how food is actually served to the user and the user's eating habits.

As shown in FIG. 1(a), although an actual menu may list stir-fried webfoot octopus, if the user is served with stir-fried webfoot octopus on top of rice, the food actually consumed by the user becomes rice with stir-fried webfoot octopus; however, if the analysis relies solely on the food menu, it may incorrectly record the user as having consumed stir-fried webfoot octopus.

Also, as shown in FIG. 1(b), although the actual menu lists pork cutlet, the user may receive pork cutlet served with pork cutlet sauce. In this case, the recorded data may indicate that the user has consumed only the pork cutlet. Since pork cutlet sauce is omitted from the food information, there may be differences between actual and analyzed calories and nutrients consumed.

Therefore, to consider all the situations above, based on a pre-stored menu, the present disclosure subdivides a food list written on the food menu to derive foods a user may additionally consume using an artificial intelligence-based machine learning model. Then, foods in a captured image are identified using the subdivided food list.

At this time, the captured image may be an image that includes the tableware, which may be an image captured through at least one image capture apparatus installed within a dining space (e.g., a cafeteria or a restaurant) in which at least one user is served with a meal, or an image captured directly by the user using the user terminal 300. However, in what follows, for the convenience of descriptions, it is assumed that the image is obtained by at least one image capture apparatus installed within the dining space in which at least one user is served with a meal.

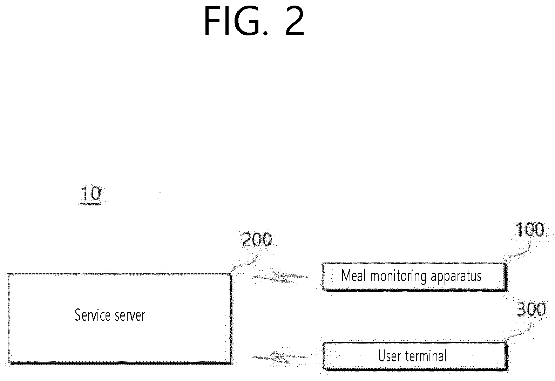

FIG. 2 illustrates a network structure of a meal monitoring system according to an embodiment of the present disclosure.

Referring to FIG. 2, a meal monitoring system 10 may comprise a meal monitoring apparatus 100, a service server 200, and a user terminal 300.

When a food menu is input, the meal monitoring apparatus 100 generates food information by subdividing the food list on a pre-stored food menu using an artificial intelligence-based machine learning model. In other words, the food information may include a subdivided food list.

Here, the food menu may include at least one food list, and a user may select and use one appropriate food list within the food menu based on date and time information.

Afterward, the meal monitoring apparatus 100 captures an image that includes a food tray (tableware) held by each user or placed before the user through at least one image capture apparatus installed inside a dining space operated based on the food menu in which at least one user is served with a meal and generates and stores meal information using the captured image and a subdivided food list.

At this time, the meal information may include information on the dining place, foods consumed, the amount consumed, the amount of leftover food, and mealtime as well as the acquired image.

The meal monitoring apparatus 100 transmits meal information to the service server 200 for analysis to provide a meal monitoring service.

When meal information is received from the meal monitoring apparatus 100, the service server 200 may analyze the meal information and upload analysis information to a platform as an analysis result of the meal information, thereby allowing the user to check the analysis information.

At this time, the analysis information may include information on the list of foods the user has consumed, amount consumed, calories consumed, nutrients consumed, food preferences, nutrients that require supplementation, and recommended nutritional supplements. Since some information may be excluded or other information may be added, the analysis information is not limited to the specific example above.

The user terminal 300 is the user's own terminal for using a meal monitoring service. At this time, the user terminal 300 may be a computer in which a plurality of application programs (i.e., applications) desired by the user are installed and executed, a ultra-mobile PC (UMPC), a workstation, a net-book, a personal digital assistant (PDA), a portable computer, a web tablet, a wireless phone, a mobile phone, a smartphone, a pad, a smart watch, a wearable terminal, an e-book, a portable multimedia player (PMP), a portable game apparatus, a navigation apparatus, a black box or a digital camera, and other mobile communication terminal. Therefore, the user terminal 300 may need to install a separate program or application to receive a meal monitoring service. In other words, the user may not only enter various pieces of information on the user to the platform through a separate program or application but also use a meal monitoring service by receiving analysis information on the user. However, the description above is only an example; thus, the user may still use a meal monitoring service by accessing a webpage.

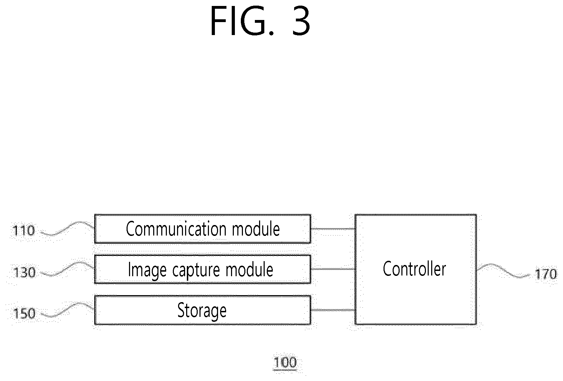

Meanwhile, FIG. 3 is one embodiment, where the meal monitoring apparatus 100 itself may be a service server 200, and in this case, the meal monitoring apparatus 100 may perform the operation of the service server 200 described above. However, in this case, image capture may be performed through a separate image capture apparatus installed within a dining space in which at least one user is served with a meal, and the captured image may be received and used immediately after the image is captured or at predetermined intervals. Also, when the user captures an image through the user terminal 300, the user may directly upload the captured image on the platform after the image capture, or the captured image including a food tray (tableware) may be made to be automatically uploaded on the platform at the time of the image capture, thereby enabling the meal monitoring apparatus 100 to receive and use the captured image.

FIG. 3 is a block diagram illustrating the structure of a meal monitoring apparatus according to an embodiment of the present disclosure.

Referring to FIG. 3, a meal monitoring apparatus 100 according to an embodiment of the present disclosure may include a communication module 110, an image capture apparatus 130, a storage 150, and a controller 170.

The communication module 110 performs communication with a user terminal, which transmits and receives a wireless signal through a communication network based on wireless Internet technologies.

Examples of the wireless Internet technologies include Wireless LAN (WLAN), Wireless- Fidelity (Wi-FI), Wireless Fidelity (Wi-Fi) Direct, Digital Living Network Alliance (DLNA), Wireless Broadband (WiBro), World Interoperability for Microwave Access (WiMAX), High Speed Downlink Packet Access (HSDPA), High Speed Uplink Packet Access (HSUPA), Long Term Evolution (LTE), and Long Term Evolution-Advanced (LTE-A); the meal monitoring apparatus 100 transmits and receives data according to at least one wireless Internet technology within its operational range, including those Internet technologies not explicitly listed above.

To support short range communication, at least one of Bluetooth, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, and Wireless Universal Serial Bus (Wireless USB) may be used. A short-range wireless area network based on at least one of the technologies above may support wireless communication between the meal monitoring apparatus 100 and the user terminal 300. At this time, the short-range wireless communication network may be a wireless personal area network.

The image capture module 130 includes at least one image capture apparatus; at least one image capture apparatus may be installed within a dining space and capture images before and after a meal while at least one user is eating. At this time, the image capture module 130 may be installed at the food serving line or on the ceiling above the table where meals are served, the installation form of which is not limited to a specific type. In other words, the image capture module 130 may be installed at any location where images of the user's dishes (food tray) may be captured. Also, the image capture module 130 may be used at different locations within the dining space according to the needs of a manager managing the dining space.

At this time, at least one image capture apparatus may include at least one of a 2D camera, a 3D camera, a Time of Flight (ToF) camera, a light field camera, a stereo camera, an event camera, an infrared camera, a lidar sensor, and an array camera; the image capture apparatus is not limited to a specific configuration as long as the apparatus is capable of measuring image information and depth information. Also, a plurality of cameras may be arranged in the stereo camera structure to obtain left and right images for implementing stereoscopic images.

The storage 150 is a module that collectively refers to various types of storage, which may store information required to perform computational operations using AI, machine learning, and artificial neural networks. The storage 150 may store various machine learning models; the machine learning models may be used to infer output from both new input data and task-related data, where the inferred values may serve as a reference for performing specific operations.

The storage 150 stores data supporting various functions of the meal monitoring apparatus 100. The storage 150 may store a plurality of application programs (or applications) executed in the meal monitoring apparatus 100 and data and commands for operating the meal monitoring apparatus 100. At least part of the application programs may be downloaded from an external server through wireless communication. Meanwhile, the application program may be stored in the storage 150, installed on the meal monitoring apparatus 100, and executed to perform an operation (function) by the controller 170.

In general, the controller 170 controls the overall operation of the meal monitoring apparatus 100 in addition to the operation related to the application program. The controller 170 may provide or process information or functions relevant to the user by processing signals, data, or information input or output through the constituting elements described above or by executing application programs stored in the storage 150.

Specifically, the controller 170 generates food information by analyzing a pre-stored food menu based on an artificial intelligence-based machine learning model; when an image including a food tray is obtained from at least one image capture apparatus, classifies at least one food item included in the captured image based on the generated food information; and generates and stores meal information based on the classified at least one food item.

In particular, when generating food information by analyzing a pre-stored food menu based on the artificial intelligence-based machine learning model, the controller 170 generates a subdivided food list as food information by extracting a food list from the pre-stored food menu and subdividing the extracted food list based on a pre-stored classification table.

Here, the pre-stored food menu may include at least one food list provided by a restaurant or a cafeteria, and each food list may arrange and list food items served at specific date and time.

At this time, since various modifiers may be applied (used) to the menu names included in the pre-stored food menu, an operation of converting the food menu to a menu consisting of common menu names based on big data and/or word embedding (word2vec) may be further performed when a food list from the pre-stored menu is extracted. Here, the common menu name may be the name of a base class included in the pre-stored classification table or a menu name corresponding to the base class (this is so because the name of the base class and the menu name corresponding to the base class may be different from each other).

Also, the pre-stored classification table is a table generated by inputting a predetermined number of images to a machine learning model and training the model to learn the respective classes of the plurality of foods and includes at least one class for identifying each of the plurality of foods. Furthermore, the pre-stored classification table may define a relationship for each of the plurality of foods as at least one of a mix class and a subclass.

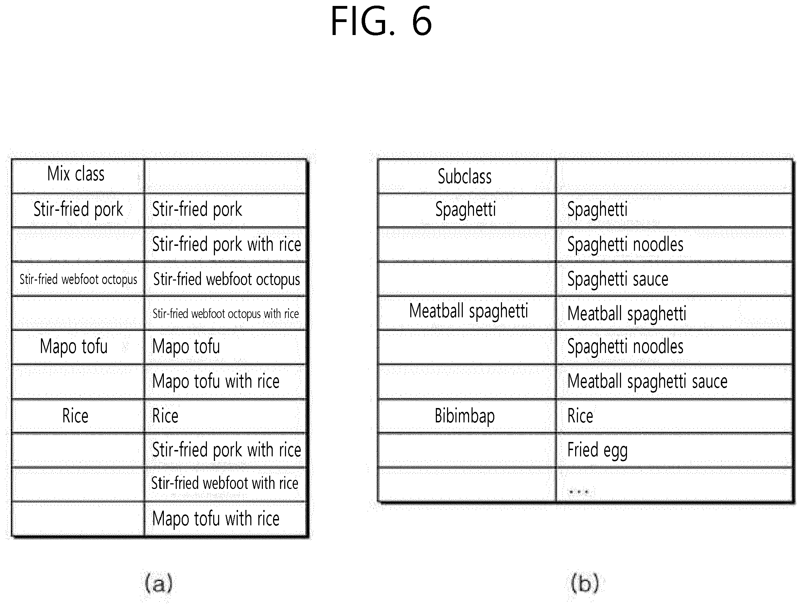

At this time, the mix class may be a class for classifying a food resulting from a combination with at least one different food, while the subclass may be a class for classifying a food which may be subdivided into at least two foods. The mix and subclasses will be described later in detail through one example with reference to FIG. 6.

In other words, the controller 170 subdivides a food list using the pre-stored classification table according to whether foods included in the food list checked from a pre-stored food menu are a mix class or a subclass. In other words, among foods included in the food list, a food belonging to the mix class is described after being subdivided into a plurality of individual foods, while a food falling under the subclass is described by adding a corresponding food alongside. In this way, a subdivided food list is obtained. At this time, the controller may further define the relationship between foods included in the extracted food list and the subdivided food list.

Meanwhile, when classifying at least one food included in a captured image based on the subdivided food list included in the food information, the controller 170 performs labeling on each food. Specifically, the controller 170 may check the class of each food based on the pre-stored classification table and tag the class on the captured image.

Afterward, the controller 170 may store the labeled image as new training data; when the amount of new training data reaches a predetermine number, the controller 170 may update the pre-stored classification table by inputting the stored new training data to the machine learning model and again performing learning of the respective classes for a plurality of foods. In other words, as a sufficient number of labeled images are collected, the existing classes included in the pre-stored classification table may also be further subdivided into classes with a smaller concept. However, those classes of small concept may be grouped into the same category depending on the needs and may be used as a higher-level concept group that collectively represents the classes of small concept.

For example, suppose a seaweed soup class exists in a pre-stored classification table; if images containing seaweed soup among labeled images reaches a predetermined number as time progresses, re-learning of the seaweed soup class is performed using the predetermined number of labeled images. In this way, the existing seaweed soup class may be further subdivided into classes of beef and seaweed soup, tuna and seaweed soup, mussel and seaweed soup, clam and seaweed soup, and sea urchin and seaweed soup.

Specifically, the controller 170 matches (checks) a class existing within a pre-stored classification table to each food extracted from the corresponding food menu; if the corresponding food is determined to be of a smaller concept than the class existing within the pre-stored classification table, the controller 170 may classify the corresponding food into the class existing within the pre-stored classification table by adding the corresponding food as a subclass of the existing class. However, the added subclass is not immediately applied to the pre-stored classification table; when the number of images corresponding to the subclass reaches a predetermined number, learning is performed based on the stored images, and then the subclass is applied to the pre-stored classification table.

At this time, when a subclass is added to the pre-stored classification table, the subclass is added so that it falls under a class with a higher-level concept than the sub-class. A plurality of subclasses may depend on the base class, which has the highest-level concept, and a subclass may also include classes with a lower-level concept, where, in this case, the number of subclasses is not limited to a specific value. Here, the base class may be a commonly used name providing the largest concept for the corresponding food.

Meanwhile, when an image is captured, the controller 170 checks the class of each menu based on a food menu corresponding to the image among pre-stored food menus and the pre-stored classification table; if a menu that does not have a matching class within the pre-stored classification table exists among the menus extracted from the corresponding food menu, the corresponding menu may be applied and used as the controller 170 updates the pre-stored classification table by adding the name of the corresponding menu to the pre-stored classification table as a base class. At this time, the added class may be immediately applied and used in one example. In another example, classes of similar menus are checked first, classification is performed based on the checked classes. If the number of images corresponding to the added class reaches a predetermined number at a later time, learning may be performed based on the stored images, and an updated classification table may then be applied and used.

As described above, if at least one class is subdivided, re-learning of the class may be performed using accumulated labeled images without involving re-labeling based on the subdivided classes.

Therefore, as time progresses, the pre-stored classification table may be subdivided further as data (images) are accumulated, leading to improved accuracy in classifying food menus.

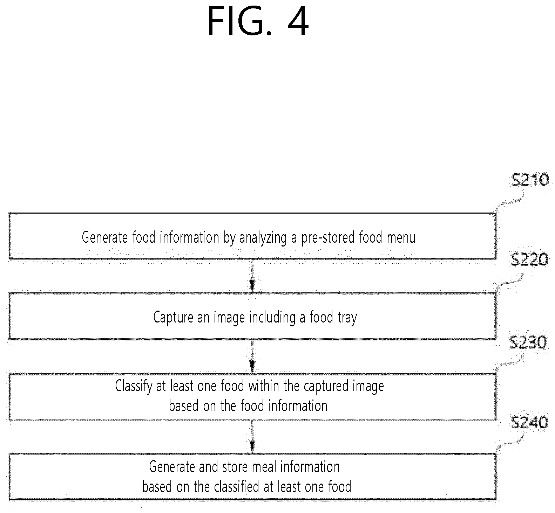

FIG. 4 is a flow diagram illustrating a meal monitoring method according to an embodiment of the present disclosure.

Referring to FIG. 4, if a manager inputs or transmits a pre-stored food menu, the meal monitoring apparatus 100 stores the food menu, analyzes the pre-stored food menu, and generates food information S210.

Afterward, the meal monitoring apparatus 100 obtains an image including a food tray S220 by capturing images of a dining space and the tableware (food tray) of at least one user having a meal within the dining space using at least one image capture apparatus and classifies S230 at least one food within the image obtained in the S220 step based on the food information generated in the S210 step. In the S220 step, image capture may be performed at least one or more times when food is served to at least one user or before and after a meal. The image capture may be set or changed by a manager, and unnecessary image capture may be avoided by setting parameters such as image capture time or the number of image captures according to the information to be analyzed.

Next, meal information on at least one user is generated and stored based on classified at least one food S240.

Meanwhile, although not shown in FIG. 4, the meal monitoring apparatus 100 may analyze meal information of at least one user and upload the analysis information of each user on a platform; when a separate service server 200 is employed, the meal monitoring apparatus 100 may transmit meal information to the service server 200 to make the service server 200 upload, on the platform, analysis information obtained by analyzing meal information of each user.

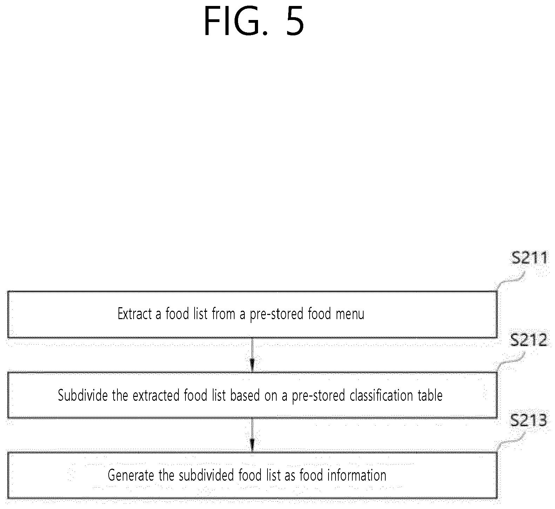

FIG. 5 illustrates a specific operation of generating food information by a meal monitoring method according to an embodiment of the present disclosure, which illustrates the S210 step of FIG. 4 in more detail.

Referring to FIG. 5, the meal monitoring apparatus 100 extracts (checks) a food list from a pre-stored food menu when generating food information S211 and subdivides the food list extracted in the S211 step based on a pre-stored classification table.

Specifically, when an extracted food list is subdivided, each food of the extracted food list is checked against the pre-stored classification table to determine whether it belongs to a mix class or a subclass; if the checking result indicates that a food correspond to a mix class, foods resulting from a combination of other foods are added to the extracted food list; and when a food corresponds to a subclass, the food is subdivided into at least two individual foods, and the subdivided foods are added to the extracted food list.

However, when extracting a food list from a pre-stored food menu, accurate extraction may be difficult because various modifiers are often used together with the corresponding menu name rather than a straightforward use of the menu name.

In this respect, a word embedding model (word2vec) may be used, for example. Word embedding is an inference-based technique that vectorizes the meaning of a word to consider the similarity between words (keywords), which quantizes text to discover knowledge from a new perspective. Through the word embedding technique, a vector containing the most comprehensive meaning of a word is generated, and the similarity or relevance between word pairs is evaluated and analyzed.

FIG. 6 illustrates one example of a mix class and a subclass that may be included in a pre-stored classification table according to one embodiment of the present disclosure.

First, the pre-stored classification table comprises classes for identifying each of a plurality of foods. At this time, a class of food that may be combined with at least one other food from among the classes to form another food is classified once again as a mix class, while a class of food that may be separated into at least two food items is classified once again as a subclass.

For example, suppose a plurality of foods include stir-fried pork, stir-fried octopus, mapo tofu, rice, spaghetti, meatball spaghetti, and bibimbap; as shown in FIG. 6, stir-fried pork, stir-fried octopus, mapo tofu, and rice may be classified as a mix class, while spaghetti, meatball spaghetti, and bibimbap may be classified as a subclass.

In other words, depending on how food is served to a user by a food service attendant, since stir-fried pork, stir-fried octopus, and mapo tofu may be provided to the user as it is or poured over rice and served in the form of stir-fried pork with rice, they are classified as a mix class (a), while spaghetti, meatball spaghetti, and bibimbap are classified as a subclass (b) because their ingredients may be served separately.

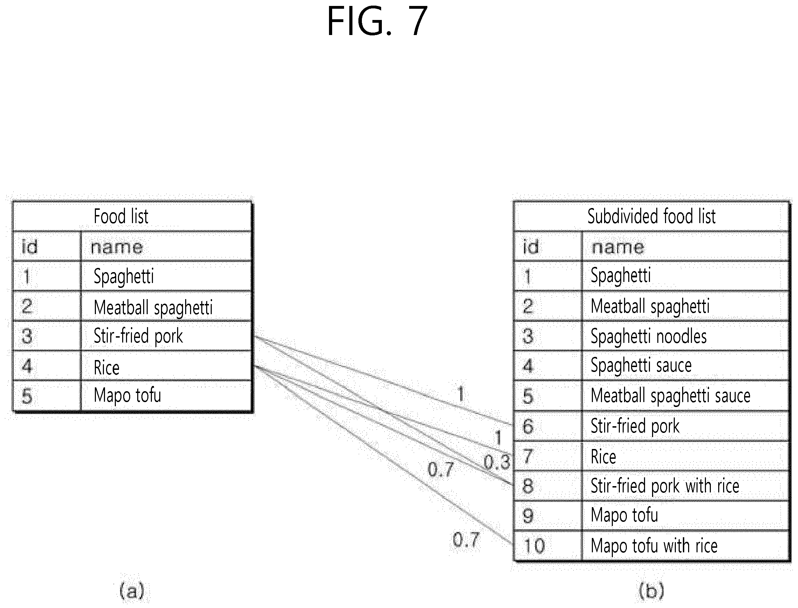

FIG. 7 illustrates the relationship between foods included in a pre-stored food menu and a subdivided food list according to one embodiment of the present disclosure.

Referring to FIG. 7, when a food list (a) extracted from a pre-stored food menu includes spaghetti, meatball spaghetti, stir-fried pork, rice, and mapo tofu, spaghetti and meatball spaghetti classified as a subclass are subdivided into spaghetti, meatball spaghetti, spaghetti noodles, spaghetti sauce, and meatball spaghetti sauce, while stir-fried pork, rice, and mapo tofu classified as a mix class is subdivided into stir-fried pork, rice, stir-fried pork with rice, mapo tofu, and mapo tofu with rice.

A subdivided food list (b) may be generated by reflecting the individual foods in the food list.

In addition, a relationship between an extracted food list and a subdivided food list may be further defined. Here, among the foods included in the related subdivided food list, at least one food item constituting a specific food included in the extracted food list is identified, and a ratio of the at least one food item identified previously to the specific food is determined.

As shown in FIG. 7, when the ratio of rice to toppings in the rice menu with toppings is set to 7:3, the proportion of rice in the stir-fried pork with rice or mapo tofu with rice may all be set to 0.7, and the portion allocated to toppings in the respective rice dishes with toppings may be determined as 0.3.

Meanwhile, although not shown in FIG. 7, for a case in which an extracted food list includes stir-fried pork but which type of rice is served is not identified, all kinds of rice determined as classes, such as polished rice, black rice, and barley rice, have to be added to the subdivided food list to open up the possibility for a different rice type.

However, as the number of types of food added to the subdivided food list increases, the number of relationships that have to be established increases. For example, as the types of rice with toppings increase and the types of rice that may be included in the food of rice with toppings also increase, the relationships that need to be established increase. Therefore, to group various types of rice into a category, all classes corresponding to the types of rice are grouped into one group. Then, several types of rice may be categorized into a group called "rice," and the relationship may be formed only once. At this time, as described above, when the amount of learning data for one class reaches a predetermined number, the group may have been subdivided through re-learning using the accumulated, stored training data.

The steps of a method or an algorithm described with respect to an embodiment of the present disclosure may be implemented directly by hardware modules or software modules executed by the hardware modules or a combination thereof. A software module may reside on a Random Access Memory (RAM), a Read Only Memory (ROM), an Erasable Programmable ROM (EPROM), an Electrically Erasable Programmable ROM (EEPROM), a flash memory, a hard disk, a removable disk, a CD-ROM, or a computer-readable recording medium of arbitrary form well known to the technical field to which the present disclosure belongs.

So far, although the embodiments of the present disclosure have been described with reference to appended drawings, it should be understood by those skilled in the art to which the present disclosure belongs that the present disclosure may be embodied in other specific forms without changing the technical principles or essential characteristics of the present disclosure. Therefore, the embodiments described above should be regarded as being illustrative rather than restrictive in every aspect.

## Claims

1. An artificial intelligence-based meal monitoring apparatus, a meal monitoring apparatus, the apparatus comprising:
a communication module;
an image capture module capturing an image including a food tray based on at least one image capture apparatus;
a storage storing various pieces of information or data, and at least one process required to monitor a meal; and
a controller performing an operation for monitoring the meal based on the at least one process,
wherein the controller generates food information by analyzing a pre-stored food menu; classifies at least one food included in a captured image based on the generated food information when the image including a food tray is obtained based on at least one image capture apparatus; and generates and stores food information based on the classified at least one food,
wherein, when the food information is generated, a food list is extracted from the pre-stored food menu; the extracted food list is subdivided based on a pre-stored classification table; and the subdivided food list is generated as food information.

2. The apparatus of claim 1, wherein the pre-stored classification table defines a relationship for each of a plurality of foods as at least one of a mix class and a subclass,
wherein the mix class is a class for classifying a food resulting from a combination of at least two foods,
while the subclass is a class for classifying a food which is added as an auxiliary food through one food.

3. The apparatus of claim 2, wherein, when the at least one food is classified, the controller classifies at least one food included in the captured image based on the subdivided food list and performs labeling on at least one food included in the captured image.

4. The apparatus of claim 1, wherein, when the food information is generated, the controller defines a relationship between the extracted food list and the subdivided food list based on correlation.

5. The apparatus of claim 4, wherein the correlation is determined by identifying at least one food constituting a specific food included in the extracted food list among those foods included in the subdivided food list and according to a proportion of the identified at least one food in the specific food.

6. The apparatus of claim 3, wherein the pre-stored classification table is generated by inputting at least one image to an artificial intelligence-based machine learning model as training data and training the machine learning model to learn the respective classes of a plurality of foods and includes at least one class for identifying each of the plurality of foods,
wherein the controller updates the pre-stored classification table by further training the machine learning model using the labeled image as the training data.

7. The apparatus of claim 6, wherein the at least one class includes at least one group including foods of the same category, and
the at least one group is further subdivided through the re-learning.

8. The apparatus of claim 1, wherein, when a food list is extracted from the pre-stored food menu, the controller converts the food menu into a common menu name based on at least one of big data and word embedding,
wherein the common menu name is a name of a base class included in the pre-stored classification table or a menu name corresponding to the base class.

9. An artificial intelligence-based monitoring method performed by a apparatus, a meal monitoring method, the method comprising:
generating food information by analyzing a pre-stored food menu;
obtaining an image including a food tray based on at least one image capture apparatus;
classifying at least one food included in the captured image based on the generated food information; and
generating and storing food information based on the classified at least one food,
wherein the generating of the food information comprises:
extracting a food list from the pre-stored food menu;
subdividing the extracted food list based on a pre-stored classification table; and
generating the subdivided food list as food information.

10. The method of claim 9, wherein the pre-stored classification table is a list of pre-learned classes for each of a plurality of foods,
each of the pre-learned classes is defined as one of a mix class and a subclass,
wherein the mix class is a class for classifying a food resulting from a combination with at least one different food,
while the subclass is a class for classifying a food which is subdivided into at least two foods.

11. The method of claim 10, wherein the classifying of the at least one food classifies at least one food included in the captured image based on the subdivided food list and performs labeling on at least one food included in the captured image.

12. The method of claim 9, wherein the generating of the food information further comprises defining a relationship between the extracted food list and the subdivided food list based on correlation,
wherein the correlation is determined by identifying at least one food constituting a specific food included in the extracted food list among those foods included in the subdivided food list and according to a proportion of the identified at least one food in the specific food.

13. The method of claim 11, wherein the pre-stored classification table is generated by inputting at least one image to an artificial intelligence-based machine learning model as training data and training the machine learning model to learn the respective classes of a plurality of foods,
includes at least one class for identifying each of the plurality of foods, and
is updated by re-training the machine learning model using the labeled image as the training data,
wherein the at least one class includes at least one group including foods of the same category, and
the at least one group is further subdivided through the re-learning.

14. The method of claim 9, wherein the extracting of the food list from the pre-stored food menu further comprises:
converting the food menu into a common menu name based on at least one of big data and word embedding,
wherein the common menu name is a name of a base class included in the pre-stored classification table or a menu name corresponding to the base class.

15. A non-transitory computer-readable recording medium being integrated with a computer and storing a computer program for operating the computer to perform a meal monitoring method of any one of claims 9 to 14.
